# EUROPÄISCHE PATENTSCHRIFT

(11) **EP 0 630 615 B1**
(45) Veröffentlichungstag und Bekanntmachung des Hinweises auf die Patenterteilung: **29.07.1998**
(21) Anmeldenummer: 94108657.1
(22) Anmeldetag: 07.06.1994
(51) Int. Cl.: A61B 17/12

(54) **Set zur Behandlung von Gefässmissbildungen**
Set for treatment of vessel deformities
Jeu pour traitement d'anomalies vasculaires

(30) Priorität: 16.06.1993 DE 4319829
(43) Veröffentlichungstag der Anmeldung: 28.12.1994
(73) Patentinhaber: Lerch, Karl-Dieter, Dr. med., D-58452 Witten (DE)
(72) Erfinder: Lerch, Karl-Dieter, Dr. med., D-58452 Witten (DE)
(74) Vertreter: Henfling, Fritz, Dipl.-Ing.

(56) Entgegenhaltungen:
- EP-A- 0 178 469
- WO-A-90/05491
- WO-A-91/08708
- DE-A- 3 044 186
- US-A- 5 201 746

## Beschreibung

Die Erfindung betrifft ein Set zur Behandlung von Gefäßmißbildungen der im Oberbegriff des Patentanspruchs 1 umrissenen Gattung. Ein solches Set ist aus WO 90/05491 zu entnehmen.

Zur Ausschaltung von Hirngefäßmißbildungen, wie Angiomen und Aneurysmen, werden seit einiger Zeit aus einer austenitischen Stahllegierung in Implantationsqualität bestehende sogenannte Aneurysma-Clips verwendet, deren Schenkel unter der Einwirkung einer dem Clip zugeordneten Feder ihre Schließ- bzw. Klemmlage einnehmen. Diese Clips werden mit Hilfe eines zangenartigen Setzwerkzeuges, mit dem die Schenkel gegen die Bestandteil des Clips bildende Feder gespreizt werden, an der Mißbildung angesetzt, wo die Schenkel des Clips unter der Einwirkung der auf sie einwirkenden Feder bei gelöstem Setzwerkzeug wieder ihre Schließ- bzw. Klemmlage, die Mißbildung totlegend, einnehmen. Clips aus einer Stahllegierung sind allerdings nicht vollständig korrisionsfest, gesetzte Clips geben vielmehr toxische Korrosionsprodukte ab, ihre Biokompatibilität ist also eingeschränkt. Eine zumindest eingeschränkte Kompatibilität besteht dann auch in Zusammenhang mit modernen bildgebenden Überwachungs- bzw. Kontrollverfahren, wie der Computertomographie und der Kernspintomographie, da das sich bei der Anwendung dieser Verfahren ausbildende Magnetfeld eine Bewegung des gesetzten Clips hervorrufen kann, die nicht nur die Abbildungsqualität negativ beeinflußt sondern darüberhinaus dann auch zu einem risikobehafteten Lösen des Clips führen kann. Des weiteren verursachen solche Clips bei der Kernspintomographie Bildartefakte, die eine Beurteilung der benachbarten Hirnstrukuren nahezu unmöglich machen. Weiterhin sind die herkömmlichen Clips nicht stereotaktisch bzw. endoskopisch applizierbar. Die Clips können dann auch nur bei offenen Hirnoperationen mit relativ breiten Zugängen eingesetzt werden, zumal die Backen des zangenartigen Setzwerkzeugs die unmittelbare Inaugenscheinnahme des Applikationsbereichs erheblich beeinträchtigen. Das aus WO 90/05491 entnehmbare Setzwerkzeug läßt sich nur in Verbindung mit Federeigenschaften aufweisenden Clips einsetzen. Das flexible Sondenrohr dieses Setzwerkzeuges erweist sich dann auch für das gezielte Ansetzen des Clips als ungeeignet, insbesondere dann, wenn eine große Genauigkeit des Ansetzens des Clips unabdingbar ist, etwa bei Manipulationen im Bereich des Gehirns.

Ausgehend von diesem Stand der Technik liegt der Erfindung das Bedürfnis nach einem Set zur Behandlung von Gefäßmißbildungen, insbesondere zur Behandlung von Mißbildungen im Bereich des Gehirns zugrunde, das die im Vorausgehenden angesprochenen Nachteile vermeidet.

Dem Bedürfnis wird mit einem gattungsgemäßen Set zur Behandlung von Gefäßmißbildungen Rechnung getragen, bei dem
die aus Titan bestehende Klammer von zwei im entlasteten Zustand gespreizten Halbrundprofilstäben gebildet wird, die fußseitig in einem am Umfang Ecken ausbildenden Sockel zusammengefaßt sind, dem ein die Stäbe einfassender, in Längsrichtung der Klammer über eine Auswölbung der Halbrundprofilstäbe verlagerbarer Klemmring vorgelagert ist, der von einem beidseitig über die Halbrundprofilstäbe der Klammer vorspringenden Dorn durchsetzt ist,
und das in der Handhabe des Setzwerkzeuges in axialer Richtung gegenüber der Handhabe verlagerbare, in der Handhabe um seine Längsachse drehbar gelagerte, biegesteife Sondenrohr mit dem sich darin führenden, gegenüber der Handhabe in axialer Richtung lagefixierten Setzstab stirnseitig eine Aufnahme für den Bestandteil der Klammer bildenden Klemmring durchsetzenden Dorn nach Art eines Bajonettverschlusses aufweist.

Ausgestaltungen des erfindungsgemäßen Sets ergeben sich aus den Unteransprüchen 2 bis 8.

Die Verwendung von Titan als Werkstoff für den Clip trägt der Forderung nach absoluter Biokompatibilität Rechnung sowie der Forderung nach uneingeschränkter Kompatibilität mit der Computertomographie und der Kernspintomographie, allerdings bedarf es einer vom Stand der Technik abweichenden, einerseits das Spreizen der Schenkel des in den Applikationsbereich zu überführenden Clips und andererseits das die Klemmung des angesetzten Clips hervorrufende Zusammenführen der Schenkel des Clips bewirkenden Lösung, nämlich einer durch das erste Merkmal des kennzeichnenden Teils des Patentanspruchs 1 definierten Ausgestaltung des Clips in Verbindung mit einem Setzwerkzeug entsprechend dem zweiten Merkmal des kennzeichnenden Teils des Patentanspruchs 1, wodurch dann auch die Möglichkeit der Entfernung des Clips nach Abschluß des Heilungsprozesses eröffnet wird.

Ein ein starres Sondenrohr aufweisendes Setzwerkzeug ist bereits der US-A-3,518,993 zu entnehmen, bei diesem Setzwerkzeug ist das Sondenrohr allerdings der Handhabe gegenüber fixiert und der sich durch das Sondenrohr erstreckende Setzstab gegenüber dem Sondenrohr in axialer Richtung verlagerbar. In der DE-B-33 27 721 ist dann auch schon eine hämostatische Klammer beschrieben, die in Klemmstellung durch einen an sie angesetzten Klemmring fixiert wird. Ist dieser Klemmring in die Klammer integriert, erweist es sich als praktisch unmöglich, die angesetzte Klammer nach Abschluß des Heilungsprozesses wieder zu lösen, was Manipulationen hiermit im Bereich des Gehirns ausschließt. In der WO 91/08708 ist u.a. eine zwei im entlastenden Zustand gespreizte, ein Halbrundprofil aufweisende Schenkel aufweisende Klammer mit einem darauf zum freien Ende der Klammer hin verlagerbaren, zum freien Ende der Klammer hin verlagert, die Schenkel in Klemmstellung überführenden Klemmring beschrieben, die dann auch aus Titan bestehen kann. Ein aus Titan bestehender hämostatischer Clip ist der US-A-5,201,746 zu entnehmen, allerdings nicht in einer Ausbildung, die sein Ansetzen mit Hilfe eines Setzwerkzeuges, speziell im Bereich des Gehirns, ermöglicht.

Eine detaillierte Beschreibung sowohl des erfindungsgemäßen Clips als auch des erfindungsgemäßen Setzwerkzeugs ist der Zeichnungsbeschreibung zu entnehmen. Bei der Verwendung einer Sonde als Setzwerkzeug bedarf es in vorteilhafter Weise vergleichsweise kleiner Zugänge zum Applikationsbereich bei unbehinderter Sichtkontrolle des Applikationsbereichs.

In der Zeichnung ist die Erfindung anhand von schematisch dargestellten Ausführungsbeispielen für den neuen Clip und ein dafür in Frage kommendes Setzwerkzeug weitergehend erläutert. Es zeigen:
- Figur 1: das teilweise aufgebrochene Setzwerkzeug in Seitenansicht, das Sondenrohr in seiner Ausgangslage,
- Figur 2: das Werkzeug in Figur 1, das Sondenrohr in seiner anderen Endlage,
- Figur 3: den neuen Clip, ebenfalls in Seitenansicht in seiner entspannten Ausgangslage,
- Figur 3a: einen der Schenkel des Clips in Stirnansicht,
- Figur 4: das stirnseitige Ende des Setzwerkzeuges in größerem Maßstab,
- Figur 5: die Vorstufe der Zuordnung des Clips in Figur 3 zum Werkzeug,
- Figur 6: eine Zwischenstufe der Zuordnung des Clips zum Werkzeug,
- Figur 7: die Endstufe der Zuordnung des Clips zum Werkzeug,
- Figur 8: den Clip in Figur 3 in appliziertem Zustand.

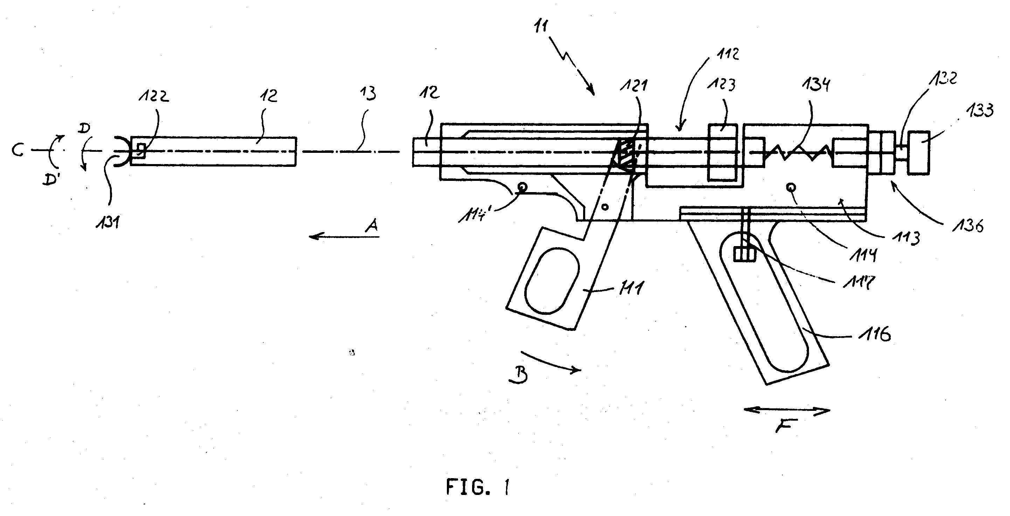

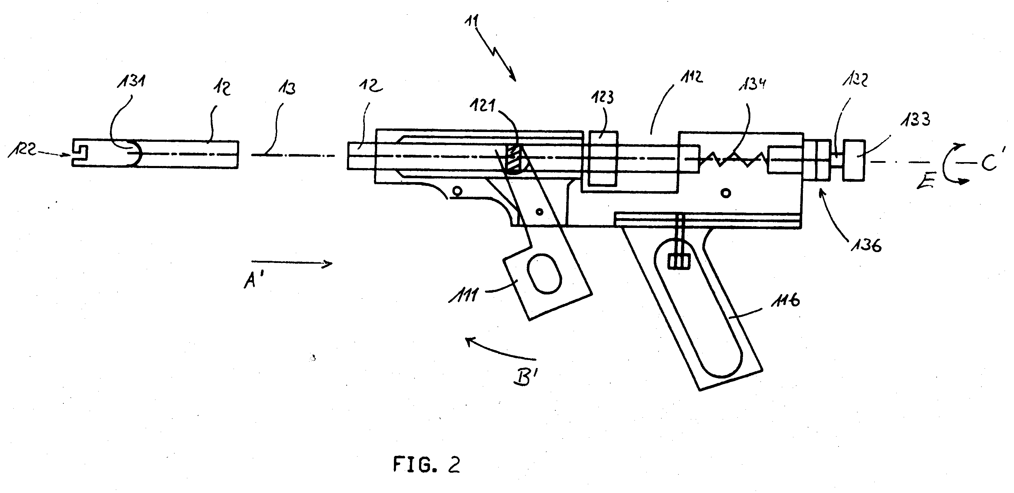

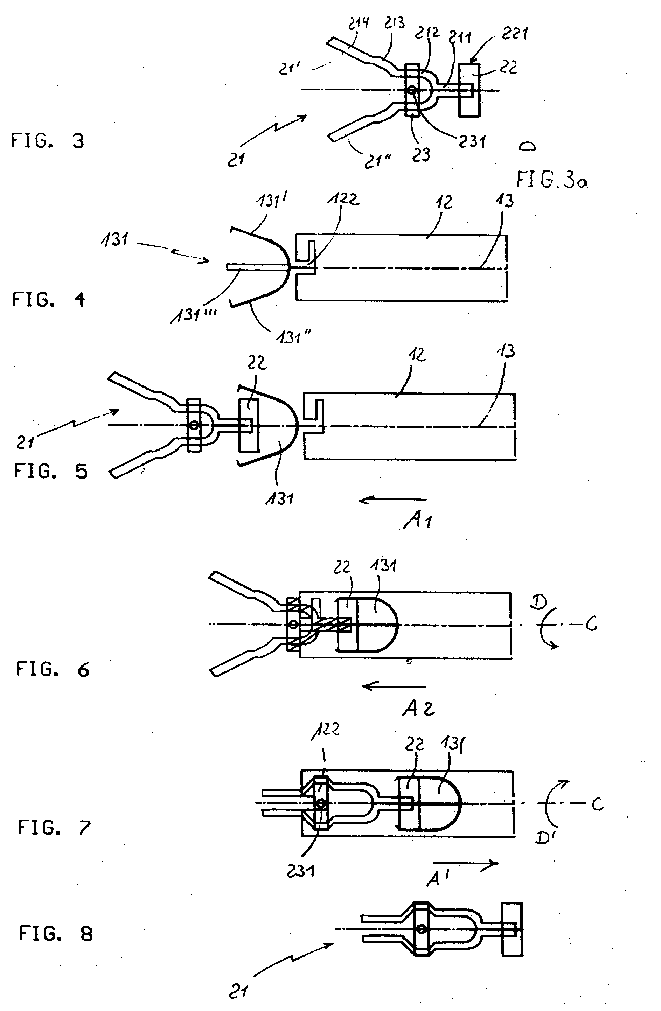

Die Figuren 1 und 2 in verkleinerter, teilweise aufgebrochener, und die Figuren 3 bis 8 in vergrößerter Darstellung.

Das in den Figuren 1 und 2 dargestellte Setzwerkzeug besteht aus dem in die nach Art des Korpus einer Pistole ausgelegte Handhabe 11 eingelagerten Sondenrohr 12, das gegenüber der Handhabe 11 im Sinne der Pfeile A bzw. A' in den Figuren 1 und 2 mit Hilfe des an einer Ringschulter 121 des Sondenrohres 12 angreifenden, im Sinne der Pfeile B bzw. B' in den Figuren 1 und 2 verschwenkbaren Betätigungsbügels 111 begrenzt verlagerbar ist. Durch das Sondenrohr 12 erstreckt sich der gegenüber der Handhabe 11 und damit gegenüber dem Sondenrohr 12 in axialer Richtung lagefixierte Setzstab 13, der am freien Ende mit einem von Klauen 131', 131'' ... gebildeten Setzkopf 131 versehen ist, dessen Klauen 131', 131'' ... sich im in Figur 2 dargestellten entspannten Zustand aufgrund ihrer Federcharakteristik in Spreizstellung befinden, aus der sie durch das über sie verlagerbare Sondenrohr 12 in die in Figur 1 dargestellte Schließstellung überführbar sind. Das Sondenrohr 12 weist dann auch noch eine von seiner Stirnseite ausgehende Aufnahme 122 auf und in Verbindung damit im Bereich der Handhabe 11, und zwar in einer das Sondenrohr 12 freilegenden Aussparung 112, einen am Umfang geriffelten Betätigungsring 123, mit Hilfe dessen das Sondenrohr 12 um seine Längsachse C im Sinne der Pfeile D bzw. D' gegenüber dem Setzstab 13 drehbar ist. Der sich durch die Handhabe 11 erstreckende Setzstab 13 ist zusammen mit dem Sondenrohr 12 mit Hilfe eines an seinem Überstand 132 über die Handhabe 11 angesetzten Betätigungsknopf 133 nach Aufheben einer durch eine Feder 134 hervorgerufenen Rastsperre 136 um seine Längsachse C' im Sinne des Doppelpfeiles E in Figur 2 drehbar. Die Handhabe 11 ist montagefreundlich aus zwei Halbschalen 113 zusammengesetzt, die durch Schrauben 114, 114' zusammengefaßt sind. Das Griffstück 116 der Handhabe 11 ist der Handhabe zwecks individueller Anpaßbarkeit im Sinne des Doppelpfeiles F verlagerbar, an der Handhabe festlegbar (117) zugeordnet. Dieses Werkzeug dient zum Setzen und gegebenfalls dann auch zum Lösen des im folgenden beschriebenen, in Figur 3 im entspannten Zustand und in Figur 8 im gespannten, also angesetzten Zustand dargestellten Clips 21 zum Totlegen von Gefäßmißbildungen.

Der mit dem in den Figuren 1 und 2 dargestellten Setzwerkzeug zu applizierende Clip 21, der in Figur 3 in entspanntem Zustand, in Figur 8 in gesetztem, also gespanntem Zustand, dargestellt ist, besteht aus zwei Schenkeln 21' und 21'' mit den Teilbereichen 211, 212, 213, 214 von denen die fußseitigen Teilbereiche 211 gegeneinander anliegend in einem Kanten 221 ausbildenden Sockel 22 zusammengefaßt sind. An das fußseitige Ende 211 der Schenkel 21', 21'' schließt sich der nach außen abgesetzte Bereich 212 an, der in die Auswölbung 213 übergeht, an die sich das gegenüber der Auswölbung 213 zurückspringende freie Ende 214 der Schenkel 21', 21'' anschließt. Fußseitig ist dem Clip 21 ein die Schenkel 21', 21'' des Clips 21 umgebender Klemmring 23 zugeordnet, der sich in seiner Ausgangslage (Fig. 3) die nach außen abgesetzten Bereiche 212 der Schenkel 21', 21'' umgibt und in seiner in Figur 7 dargestellten Klemmlage über die Auswölbungen 213 der Schenkel 21', 21'' verlagert, die im gesetzten Zustand des Clips 21 einen der Gefäßmißbildungen vorgelagerten durchbluteten Gewebebereich einfassenden und die Gefäßmißbildungen auf diese Weise abklemmenden Enden 214 der Schenkel 21', 21'' zusammenpreßt. Durchsetzt ist der Klemmring 23 mit beidseitigem Überstand von einem Dorn 231. Der in der Zeichnung größer dargestellte Abstand zwischen den Bereichen 212 der Schenkel 21', 21'' des Clips 21 ist in natura so ausgelegt, daß der den Klemmring 23 durchsetzende Dorn 231 bei der Überführung des Klemmringes 23 in die in Figur 3 dargestellte Ausgangslage die Spreizung der Schenkel 21', 21'' unterstützt bzw. bewirkt. Gleichermaßen ist der Rücksprung der freien Enden 214 der Schenkel 21', 21'' des Clips 21 so bemessen, daß die Schenkelenden im gespannten Zustand in natura einen wesentlich geringen Zwischenraum zwischen sich ausbilden als in Figur 8.

Der entspannte Clip (Fig. 3) wird am Setzwerkzeug, speziell am Kopf 131 des Setzstabes 13 bei gegenüber dem Setzkopf zurückverlagertem Sondenrohr 12 (Figuren 2 und 4) durch Zuordnung des Sockels 22 des Clips 21 zu den gespreizten Klauen 131', 131'' ... des Setzkopfes 131 angesetzt (Figur 5). Anschließend wird das Sondenrohr 12 im Sinne des Pfeiles A1 in Figur 5 durch Anziehen des Betätigungsbügels 111 gegen den Handgriff 116 der Handhabe 11 im Sinne des Pfeiles B in Figur 1 verlagert, wobei das sich über den Setzkopf 131 des Setzstabes 13 schiebende Sondenrohr 12 die zunächst gespreizten Klauen 131', 131'' ... des Setzkopfes 131 gegen den Sockel 22 des Clips 21, den Sockel 22 einfassend drückt (Figur 6). Bei weiterer Verlagerung des Sondenrohres 12 im Sinne des Pfeiles A2 in Figur 6 gelangt der den dem Clip 21 zugeordneten Klemmring 23 durchsetzende Dorn 231 in die stirnseitige Aufnahme 122 im Sondenrohr 12. Durch anschließende Drehung des Sondenrohres 12 im Sinne des Pfeiles D in den Figuren 1 und 6 gegenüber dem Setzstab 13 wird der Klemmring 23 durch den ihn durchsetzenden Dorn 231 in der stirnseitigen Aufnahme 122 des Sondenrohres 12 festgelegt. In der zuletzt beschriebenen, ein Zwischenstadium zwischen den Situationen nach den Figurn 6 und 7 bildenden Situation wird der Clip 21 mit dem Setzwerkzeug durch eine Öffnung in das Innere des Körpers in den Bereich überführt, in dem der Clip 21 angesetzt werden soll. Im gezielt angesetzten Zustand des Clips 21 wird das Sondenrohr 12 sodann weiter im Sinne des Pfeiles A2 in Figur 6 verschoben, wobei der gegenüber dem Sondenrohr 12 fixierte Klemmring 23 aus der in Figur 6 dargestellten Ausgangslage in die in Figur 7 dargestellte Klemmlage überführt wird, in der der Klemmring 23 über die Auswölbungen 213 der Schenkel 21', 21'' des Clips 21 verschoben die die Gefäßmißbildung abklemmenden Schenkelenden 214 zusammenpreßt.

Damit ist der eigentliche Setzvorgang beendet und das Setzwerkzeug wird nach Lösen des den Klemmring 23 durchsetzenden Dorns 231 aus der stirnseitigen Aufnahme 122 des Sondenrohres 12 durch Zurückdrehen des Sondenrohres 12 gegenüber dem Setzstab 13 im Sinne des Pfeiles D' in Figur 7 und anschließende Rückverlagerung des Sondenrohres im Sinne des Pfeiles A' in Figur 7 von dem gesetzten Clip 21 abgezogen. Der gesetzte Clip 21 ist im Prinzip in Figur 8 dargestellt. In natura ist der Clip so ausgebildet, daß die Schenkelenden 214 im gesetzten Zustand den der totzulegenden Mißbildung vorgelagerten Gewebebereich klemmend einfassend gegeneinander anliegen.

Wenn sich das aus operativen Gründen als erforderlich erweist, kann vorweg jedoch auch schon die Situation gemäß Figur 7 herbeigeführt werden und der in dieser Situation in den Applikationsbereich überführte Clip sodann vor dem eigentlichen Ansetzen des Clips 21 zunächst noch einmal in eine Zwischensituation überführt werden, in der der Clip 21 mit noch gespreizten Schenkeln 21', 21'' angesetzt und sodann endgültig appliziert wird.

Wie aus Figur 3a zu entnehmen ist, bestehen die Schenkel 21', 21'' des Clips 21 aus einem Halbrundprofil, dessen einander zugewandte Flachseiten im Bereich der Schenkelenden 214 zweckmäßigerweise aufgerauht bzw. geriffelt sind. Die Schenkelenden 214 können dann auch ausgehend von den Auswölbungen 213 anstelle eines gestreckten Verlaufs einen gebogenen oder aber auch einen abgewinkelten Verlauf nehmen, wenn das der Applikationsbereich erforderlich erscheinen läßt.

Bei nur vorübergehender Applikation des Clips 21, wird der Clip 21 bei entsprechender gegenläufiger Handhabung des Setzwerkzeuges wieder aus dem Applikationsbereich entfernt.

## Patentansprüche

1. Set zur Behandlung von Gefäßmißbildungen, bestehend aus einer die Mißbildung totlegenden, mit gespreizten Schenkeln an der Mißbildung ansetzbaren Klammer (21) aus körperverträglichem Material und einem Setzwerkzeug, bestehend aus einem in einer Handhabe (11) mittels eines Stellgliedes (111) in axialer Richtung verlagerbaren Sondenrohr (12) und einem sich durch das Sondenrohr (12) erstreckenden, in einen an der Klammer (21) ansetzbaren Greifer (131) in Gestalt von außer Eingriff des Sondenrohres (12) gespreizten und vom Sondenrohr (12) eingefaßt aufeinanderzuverlagerten, an der Klammer (21) wirksam werdenden Klauen (131'...) auslaufenden, in der Handhabe gelagerten Setzstab (13), dadurch gekennzeichnet, daß
die aus Titan bestehende Klammer (21) von zwei im entlasteten Zustand gespreizten Halbrundprofilstäben (21', 21'') gebildet wird, die fußseitig (211) in einem am Umfang Ecken (221) ausbildenden Sockel (22) zusammengefaßt sind, dem ein die Stäbe (21', 21'') einfassender, in Längsrichtung der Klammer (21) über eine Auswölbung (213) der Halbrundprofilstäbe (21', 21'') verlagerbarer Klemmring (23) vorgelagert ist, der von einem beidseitig über die Halbrundprofilstäbe (21', 21'') der Klammer (21) vorspringenden Dorn (231) durchsetzt ist,
und das in der Handhabe (11) des Setzwerkzeuges in axialer Richtung gegenüber der Handhabe (11) verlagerbare, in der Handhabe (11) um seine Längsachse drehbar gelagerte, biegesteife Sondenrohr (12) mit dem sich darin führenden, gegenüber der Handhabe (11) in axialer Richtung lagefixierten Setzstab (13) stirnseitig eine Aufnahme (121) für den Bestandteil der Klammer (21) bildenden Klemmring (23) durchsetzenden Dorn (231) nach Art eines Bajonettverschlusses aufweist.

2. Set nach Anspruch 1, gekennzeichnet durch eine Handhabe (11) nach Art eines Pistolenkorpus mit einem Griffstuck (116) und einem Stellglied (111) für das Sondenrohr (12) nach Art eines Abzugsbügels.

3. Set nach Anspruch 2, dadurch gekennzeichnet, daß das Sondenrohr (12) mit einer Ringschulter (121) versehen ist, die vom gabelförmig ausgebildeten Ende des Abzugsbügels (111) eingefaßt ist.

4. Set nach einem der Ansprüche 1 bis 3, dadurch gekennzeichnet, daß Sondenrohr (12) und Setzstab (13) gemeinsam gegenüber der Handhabe (11) um ihre Längsachse drehbar, feststellbar gelagert sind.

5. Set nach Anspruch 4, gekennzeichnet durch eine Feder-Rastlagerung (134, 136) des Sondenrohres (12) gegenüber der Handhabe (11).

6. Set nach einem der Ansprüche 1 bis 5, dadurch gekennzeichnet, daß die Handhabe (11) eine das Sondenrohr (12) freilegende Aussparung (112) aufweist und dem Sondenrohr (12) im Bereich dieser Aussparung (112) ein Stellglied (123) zum Verdrehen des Sondenrohres (12) gegenüber dem Setzstab (13) zugeordnet ist.

7. Set nach Anspruch 6, gekennzeichnet durch ein dem Sondenrohr (12) zugeordneten Rändelring als Stellglied.

8. Set nach einem der Ansprüche 1 bis 7, gekennzeichnet durch einen gegenüber den das Sondenrohr (12) einfassenden, durch Schraubverbindungen oder dergleichen zusammengefaßten Lagerschalen (113) in Längsrichtung verlagerbaren, feststellbaren Handgriff (116).

## Claims

1. Set for the treatment of vascular deformities, comprising a clamp (21) formed from body-compatible material, which clamp immobilises the deformity and is attachable to the deformity by means of splayed portions, and a positioning tool, comprising a tubular probe (12), which is displaceable in a handle (11) in the axial direction by means of a control member (111), and a positioning rod (13) extending through the tubular probe (12) and terminating in a gripping device (131), which is attachable to the clamp (21), in the form of claws (131'...), which are splayed out of engagement with the tubular probe (12) and displaced towards one another enclosed by the tubular probe (12), said claws becoming effective on the clamp (21), and said positioning rod being mounted in the handle, characterised in that the clamp (21), which is made from titanium, is formed from two semicircular profile rods (21', 21''), which are splayed in the unloaded condition and assembled at the bottom ends (211) in a socket (22) forming corners (221) on the periphery, in front of which socket is situated a clamping ring (23), which encloses the rods (21', 21'') and is displaceable in the longitudinal direction of the clamp (21) via an arched portion (213) of the semicircular profile rods (21', 21''), said clamping ring being traversed by a mandrel (231) protruding on both sides beyond the semicircular profile rods (21', 21'') of the clamp (21), and the bend-resistant tubular probe (12), which is displaceable in the handle (11) of the positioning tool in the axial direction relative to the handle (11) and is mounted in the handle (11) so as to be rotatable about its longitudinal axis, is provided with the positioning rod (13), which extends therein and is positionally secured relative to the handle (11) in the axial direction, and said probe has, at its front end, a receiving means (121) for the mandrel (231), which traverses the clamping ring (23) forming a component part of the clamp (21), in the form of a bayonet-type securing means.

2. Set according to claim 1, characterised by a handle (11) in the form of a pistol body with a gripping stock (116) and a control member (111) for the tubular probe (12) in the form of a trigger guard.

3. Set according to claim 2, characterised in that the tubular probe (12) is provided with an annular shoulder (121), which is enclosed by the fork-shaped end of the trigger guard (111).

4. Set according to one of claims 1 to 3, characterised in that tubular probe (12) and positioning rod (13) are mounted together so as to be securable relative to the handle (11) and rotatable about its longitudinal axis.

5. Set according to claim 4, characterised by a resilient locking arrangement (134, 136) for the tubular probe (12) relative to the handle (11).

6. Set according to one of claims 1 to 5, characterised in that the handle (11) has a recess (112) exposing the tubular probe (12), and a control member (123) is associated with the tubular probe (12) in the region of this recess (112) for rotating the tubular probe (12) relative to the positioning rod (13).

7. Set according to claim 6, characterised by a knurled ring, which is associated with the tubular probe (12) and serves as the control member.

8. Set according to one of claims 1 to 7, characterised by a securable hand grip (116) displaceable in the longitudinal direction relative to the bearing shells (113), which enclose the tubular probe (12) and are assembled by screw connections or the like.

## Revendications

1. Ensemble de traitement des malformations des vaisseaux, constitué d'une agrafe (21) pouvant être appliquée sur la malformation lorsque les branches sont écartées, fermant le site de malformation, agrafe constituée d'un matériau compatible pour le corps et d'un outil de placement, constitué d'un tube de sonde (12), déplaçable en direction axiale dans une poignée (11) au moyen d'un organe de réglage (111), et d'une barre de placement (13), montée dans la poignée, s'étendant à travers le tube de sonde (12), en allant former un organe de préhension (131), pouvant être appliqué sur l'agrafe (21) et se présentant sous la forme do griffes (131' ...), entrant en action sur l'agrafe (21), griffes écartées l'une de l'autre lorsque le tube de sonde (12) est hors de prise et déplacées l'une vers l'autre par enchâssement, sous l'action du tube de sonde (12), caractérisé en ce que
l'agrafe (21) réalisée en titane est constituée par deux barres profilées demi-rondes (21', 21'') écartées lorsqu'elles se trouvent à l'état déchargé, qui sont regroupées côté pied (211) en un socle (22) constituant des angles (221) à la périphérie, socle à l'avant duquel est monté un anneau de serrage (23) enchâssant les barres (21', 21''), déplaçable dans la direction longitudinale de l'agrafe (21) sur un bombement (213) des barres profilée demi-rondes (21', 21''), anneau de serrage traversé par un téton (231) faisant saillie des deux côtés sur les barres profilées demi-rondes (21', 21'') de l'agrafe (21),
et le tube de sonde (12) présentant une rigidité en flexion, monté avec possibilité de rotation autour de son axe longitudinal dans la poignée (11), de façon déplaçable en direction axiale par rapport à la poignée (11), dans la poignée (11) de l'outil de placement, présente avec la barre de placement (13) à position fixe en direction axiale par rapport à la poignée (11) et guidée à l'intérieur du tube de sonde (12), frontalement, un logement (121) destiné au téton (231) traversant l'anneau de serrage (23) faisant partie de la pince (21), à la façon d'un joint à baïonnette.

2. Ensemble selon la revendication 1, caractérisé par une poignée (11) réalisée à la façon d'un corps de pistolet, avec une pièce de saisie (116) et un organe de réglage (111), destiné au tube de sonde (12), réalisé à la façon d'une sous-garde ou pontet.

3. Ensemble selon la revendication 2, caractérisé en ce que le tube de sonde (12) est pourvu d'un épaulement annulaire (121) enchâssé par l'extrémité, à réalisation en forme de fourche, de la sous-garde.

4. Ensemble selon l'une des revendications 1 à 3, caractérisé en ce que le tube de sonde (12) et la barre de placement (13) sont montés de façon à pouvoir être fixés conjointement par rapport à la poignée (11), avec possibilité de tourner autour de leur axe longitudinal.

5. Ensemble selon la revendication 4, caractérisé par un montage à encliquetage et ressort (134, 136) du tube de sonde (12) par rapport à la poignée (11).

6. Ensemble selon l'une des revendications 1 à 5, caractérisé en ce que la poignée (11) présente un évidement (112) dégageant le tube de sonde (12) et un organe de réglage (123), destiné à faire tourner le tube de sonde (12) par rapport à la barre de placement (13), étant associé au tube de sonde (12) dans la zone de cet évidement (112).

7. Ensemble selon la revendication 6, caractérisé par une bague moletée, associée au tube de sonde (12), à titre d'organe de réglage.

8. Ensemble selon l'une des revendications 1 à 7, caractérisé par une poignée (116), pouvant être fixée, déplaçable en direction longitudinale par rapport aux coques de palier (113) enchâssant le tube de sonde (12) et assemblées à l'aide de liaisons vissées ou analogues.
